# EUROPEAN PATENT SPECIFICATION

(11) **EP 0 724 648 B1**
(45) Date of publication and mention of the grant of the patent: **02.04.2003**
(21) Application number: 94927989.7
(22) Date of filing: 01.09.1994
(51) Int. Cl.: C12N 15/00, C12Q 1/68

(54) **METHODS FOR SCREENING ANTIESTROGEN COMPOUNDS**
METHODEN ZUM AUFSPÜREN VON ANTI-ÖSTROGENVERBINDUNGEN
PROCEDES DE DETECTION DE COMPOSES ANTI- OESTROGENES

(30) Priority: 01.09.1993 US 115161
(43) Date of publication of application: 07.08.1996
(73) Proprietor: THE REGENTS OF THE UNIVERSITY OF CALIFORNIA, Oakland, California 94612-3550 (US)
(72) Inventor: KUSHNER, Peter, San Francisco, CA 94122 (US); WEBB, Paul, San Francisco, CA 94122 (US)
(74) Representative: Thiel, Christian, Dr. Dipl.-Chem.
(86) International application number: US9409898
(87) International publication number: WO95006754

(56) References cited:
- THE MERCK INDEX, 11TH EDITION 1989, MERCK & CO., INC., RAHWAY, N.J., USA, page 686 XP002009518 "4281. Genistein."
- THE MERCK INDEX 11TH EDITION 1989, MERCK & CO., RAHWAY, N.J., USA, page 583 XP002009519 "3653. Estradiol."
- PROC. NATL.ACAD SCI., vol. 87, September 1990, NATL. ACAD SCI.,WASHINGTON,DC,US;, pages 6883-6887, XP002009520 S.E. FAWELL ET AL.: "Inhibition of estrogen receptor-DNA binding by the "pure" antiestrogen ICI 164,384 appears to be mediated by impaired receptor dimerization"
- EMBO J., vol. 9, no. 9, 1990, OXFORD UNIVERSITY PRESS,GB;, pages 2811-2818, XP002009521 M. BERRY ET AL.: "Role of the two activating domains of the oestrogen receptor in the cell-type and promoter-context dependent agonistic activity of the anti-oestrogen 4-hydroxytamoxifen"
- JOURNAL OF BIOLOGICAL CHEMISTRY, Volume 268, No. 19, issued 05 July 1993, A. PHILIPS et al., "Estradiol Increases and Antiestrogens Antagonize the Growth Factor-Induced Activator Protein-1 Activity in MCF7 Breast Cancer Cells Without Affecting C-Fos and C-Jun Synthesis", pages 14103-14108.
- BIOTECHNIQUES, Volume 9, No. 4, issued 1990, M. PONS et al., "A New Cellular Model of Response to Estrogens: A Bioluminescent Test to Characterize (Anti)Estrogen Molecules", pages 450-459.
- CELL, Volume 63, issued 21 December 1991, M.-P. GAUB et al., "Activation of the Ovalbumin Gene by the Estrogen Receptor Involves the Fos-Jun Complex", pages 1267-1276.
- MOLECULAR ENDOCRINOLOGY, Volume 4, No. 10, issued 1990, P.J. KUSHNER et al., "Construction of Cell Lines that Express High Levels of the Human Estrogen Receptor and are Killed by Estrogens", pages 1465-1473.

## Description

### BACKGROUND OF THE INVENTION

Many breast tumors require estrogens for tumor growth. Thus, treatment with antiestrogen compounds can slow or prevent tumor spread. Many antiestrogens however, show both estrogen antagonistic and agonistic activity. The nonsteroidal antiestrogen tamoxifen, for example, which is established as the treatment of choice for the endocrine therapy of advanced breast cancer, shows both agonistic and antagonistic activity. Sutherland, S. & Jackson, M. *Cancer Treat. Revs.* 15: 183-194 (1987).

The agonistic activity of tamoxifen and other antiestrogens may have profound effects upon patients. For example, agonistic activity may have beneficial effects, such as preventing osteoporosis and reducing serum cholesterol. Love, *et al. New Eng. J. Med.* 326: 852-856 (1992). Love, *et al. J. Natl. Cancer Inst.* 82: 1327-1332 (1990). Conversely, agonistic activity may also be harmful. Tamoxifen for example sometimes increases endometrial tumor incidence Iino, *et al. Cancer Treat. & Res.* 53: 228-237 (1991) or switches from inhibition to stimulation of estrogen dependent growth in breast tumor progression. Parker, M.G. (ed) Cancer Surveys 14: Growth Regulation by Nuclear Hormone Receptors. Cold Spring Harbor Laboratory Press (1992).

It is desirable to identify pure antiestrogens as they are anticipated to provide more rapid, complete or longer-lasting tumor responses. Wakeling, A. E. *Breast Cancer Res. & Treat.* 25: 1-9 (1993). For example, ICI 164,384, thought to be a pure antiestrogen, blocked MCF-7 cell invasion activity of a re-constituted basement membrane while estradiol and 4'-hydroxytamoxifen stimulated this activity suggesting that early treatment of breast cancer with a pure antiestrogen might be particularly beneficial in limiting tumor spread. Braacke, *et al., Br. J. Cancer* 63: 867-872 (1991).

Conversely, while pure antiestrogens appear preferable for cancer treatments, mixed agonist-antagonist compounds may be preferable for preventative treatment. Such compounds should combine sufficient antagonist activity on estrogen stimulated breast tumor growth while maintaining simultaneous agonist activity on bone density and serum lipid levels.

Currently, antiestrogen compounds are screened with animal models such as the rat uterine test. These tests are cumbersome, slow, expensive and of uncertain application to humans because of differences between the human and rodent estrogen receptors.

Alternatively, investigators have used human breast cancer cell lines and assayed their growth as stimulated by estrogens or alternatively used the response of native or transfected reporter genes as assays of estrogen response. These cell lines utilize reporter genes that indicate activity of antiestrogen compounds that is mediated through the classical estrogen response element.

The prior art fails to provide methods for quickly and easily testing potential antiestrogen compounds for agnostic as well as antagonistic properties mediated through pathways other than the classical estrogen response pathway, that may affect, adversely or beneficially, their use in various therapeutic applications. This invention addresses these and other problems in the art.

### SUMMARY OF THE INVENTION

The present invention provides methods for screening test compounds for the ability to activate or inhibit transcription through an indirect estrogen response or classical estrogen response. The indirect estrogen response is mediated by promoters comprising an AP1 site and the classical estrogen response is mediated by promoters comprising a classical estrogen response element. Preferred AP1 sites can be isolated from metalloprotease genes. Preferred classical estrogen response elements can be isolated from the *Xenopus* vitellogenin A2 gene.

The methods typically use cells comprising an estrogen receptor and a promoter comprising an AP1 site which regulates expression of a reporter gene. The cells are then contacted with the test compound and the expression of the reporter gene is detected.

Cells used in the assay can be any cell which naturally expresses estrogen receptors or as the result of a transgene encoding the receptor. Preferred cells include MCF-7, ERC1, ERC2 or ERC3. The reporter genes used to detect an estrogen response include genes encoding beta-galactosidase and bacterial chloramphenicol acetyl transferase. The promoters used may be those which naturally comprise AP1 or estrogen receptor elements or the promoters may be genetically engineered to comprise those elements.

The assays may be used with cells comprising promoters with an AP1 site. or alternatively a second set of cells with a promoter comprising a classical estrogen response element which regulates expression of a second reporter gene can be used. Alternatively, the two promoters and reporter genes can be in the same cells. In these assays the ability of test compound to induce or inhibit both the indirect and classical pathways can be determined.

When the methods are used to identify estrogen antagonists, the test compounds are contacted with the cells and a compound known to mediate an indirect estrogen response. The ability to inhibit the response is determined by detecting the expression of the reporter gene. Compounds known to mediate an indirect estrogen response include tamoxifen and estrogen at half maximal concentrations. The compounds can also be tested for the ability to induce or block the classical estrogen pathway, as well.

The invention further provides compounds identified by the claimed methods. Also mentioned are cells which overexpress an estrogen receptor and comprise a promoter comprising an AP1 site which regulates expression of a reporter gene. Exemplary cells include ERC1.

### BRIEF DESCRIPTION OF TEE DRAWINGS

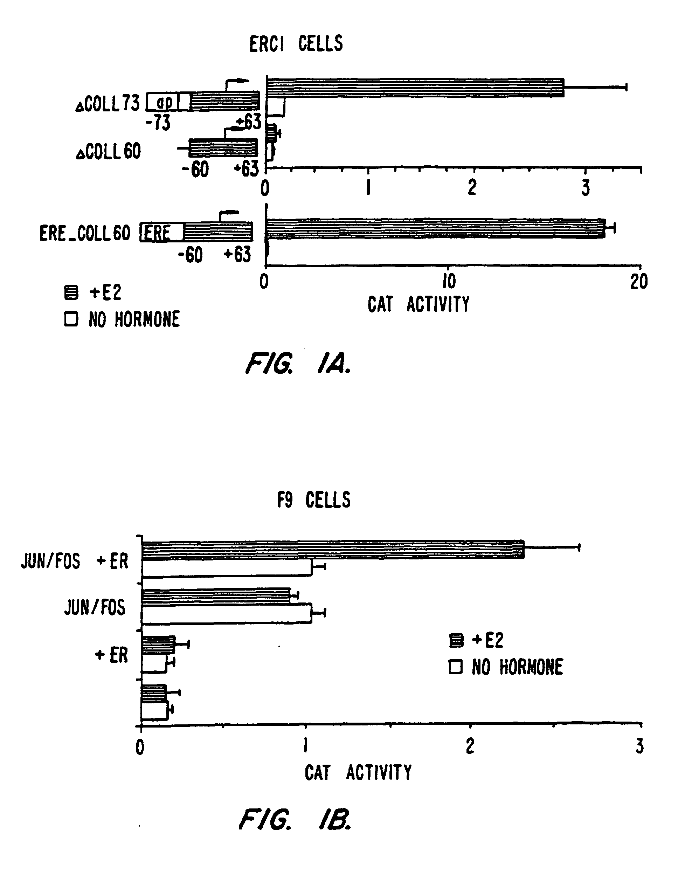

Figure 1 shows estrogen stimulation mediated by an AP1 site. (A) A consensus AP1 site has ERE-like activity. Reporter genes, Δcoll73 and Δcoll60, were transfected into ERC1 cells (Kushner, *et al.*, *Mol. Endocrinol*., 4: 1465-1473 (1990) Δcoll73 consists of human collagenase promoter (-73 to +63) cloned upstream of the CAT gene. Angel, *et al. Mol. Cell, Biol*., 7: 2256-2266 (1987). The position of the consensus AP1 site is indicated. Δcoll60 lacked the AP1 site (-73 to -60). EREΔcoll60 contains an oligonucleotide corresponding to the frog vitellogenin A2 consensus ERE (Parker, M.G. (Ed), *Cancer Surveys* 14 Growth Regulation by Nuclear Hormone Receptors. Cold Spring Harbor Laboratory Press (1992)) immediately upstream of the collagenase sequences in Δcoll60. Typical results of CAT assays, normalized for transfection efficiency, following a single transfection are shown opposite. Each point is the mean value of triplicate assays, with standard errors. CAT activities from cells maintained in the absence of hormone are shown as white bars, those in the presence of a saturating concentration (100 nM) of estradiol as black bars. (B) Estrogen induction at the AP1 site requires AP1 proteins. Results of CAT assays prepared from F9 cells transfected with Δcoll73.

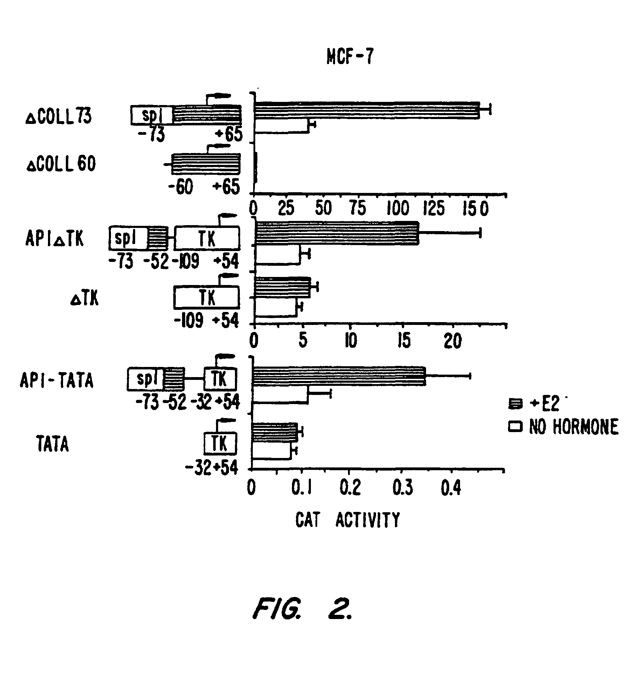

Figure 2 shows activity of AP1 sites in MCF-7 cells. Estrogen induction of AP1 dependent reporter genes Δcoll73 and Δcoll60 was described above. AP1ΔTK-CAT consists of the collagenase AP1 site cloned upstream of a herpes simplex virus TK promoter (-32 to +45). TK-TATA lacks any known upstream transcription factor binding sites. CAT activities in the presence (black) and absence (white) of estrogen are shown opposite each reporter.

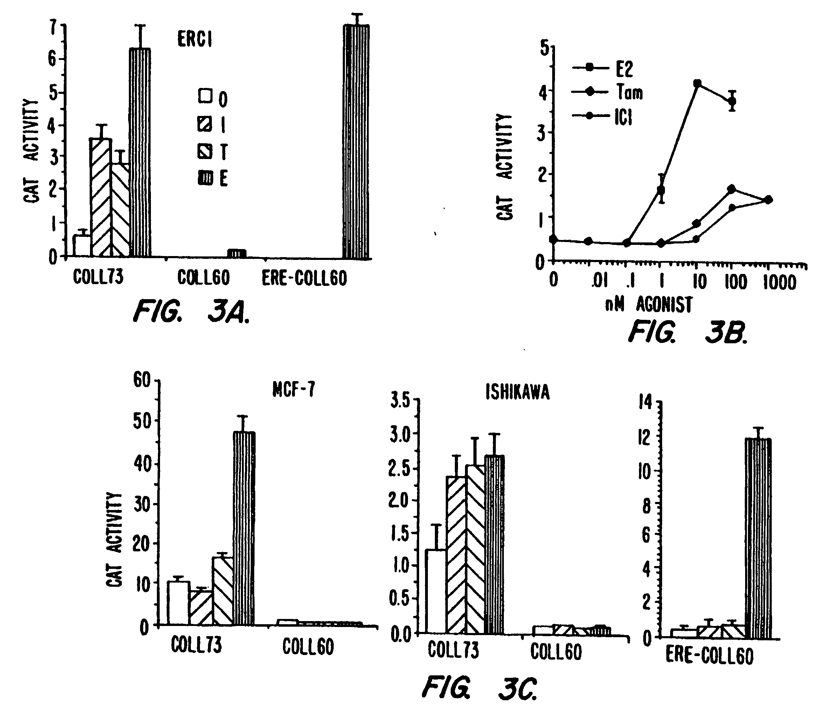

Figure 3 shows agonism by antiestrogens at the AP1 site. (A) CAT assays of ERC1 cells transfected with Δcoll73, Δcoll60 or EREΔcoll60 and incubated with saturating concentrations (100nM) of estrogen (black), tamoxifen (shaded) and ICI (striped) or no hormone (white). A single experiment is shown, where the activities are the mean of three separate points. (B) Dose dependence of antihormone agonism. ERC1, transfected with Δcoll73 were plated and exposed to a range of hormone concentrations. Each point is the mean of triplicate assays of CAT reactivity. (C) Antiestrogen activity in estrogen responsive cell lines. Results of representative CAT assays following transfection of Δcoll73 and Δcoll60 into MCF-7 cells and Δcoll73, Δcoll60 and EREΔcoll60 into Ishikawa cells are shown. A single experiment is shown, where the activities are the mean of three separate points.

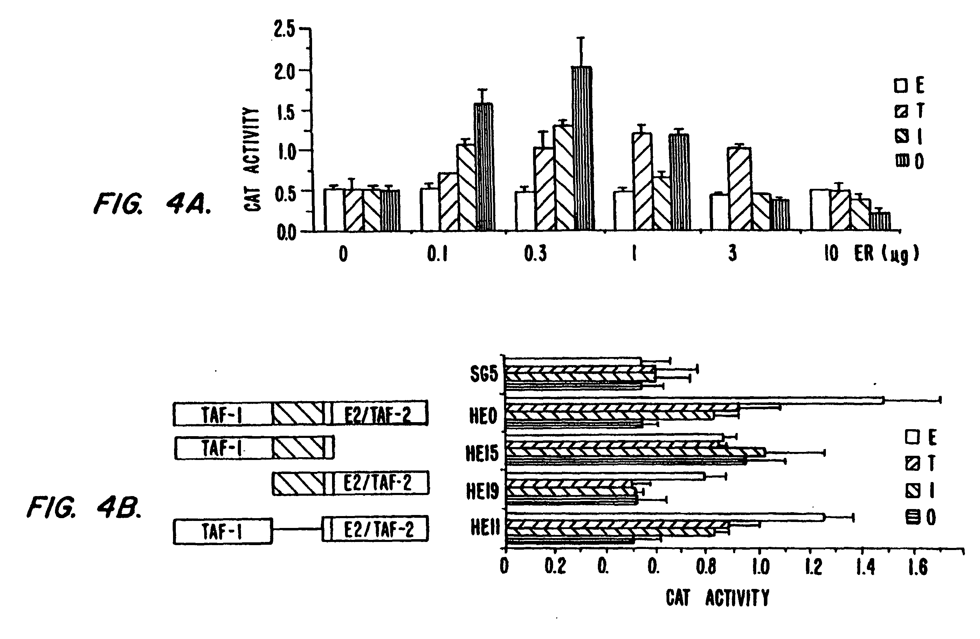

Figure 4 shows antiestrogen action in transient transfections. (A) Δcoll73 was introduced into CHO cells, with varying quantities of ER expression vector HEO (Holinka, C.F. *et al., J. Steroid Biochem.*, 25: 781-786 (1986), normalized to 10µg with the parent expression vector, SG5. CAT activities were determined in the presence of hormones, as in Figure 3A. (B) Requirement of ER domains for hormone action. The structure of four derivatives of the human ER cDNA is shown schematically. The DNA binding domain is indicated with the striped box, the ligand binding domain is marked E2. Transactivation functions TAF-1 and TAF-2 are marked. The right panel shows the effect of hormone upon Δcoll73 in the presence of each mutant. Transfections were carried out as described by Kushner, *et al*., 4: 1465-1473 (1990), in CHO cells, including 300ng of each ER expression vector.

### DESCRIPTION OF THE PREFERRED EMBODIMENT

The present invention provides an efficient way to screen large numbers of test compounds for those which have desirable properties for either the treatment or the prevention of various cancers (e.g. breast cancer, ovarian cancer, endometrial cancer) and other diseases (e.g. endometriosis) mediated by estrogen. The invention allows for screening of test compounds for agonistic as well as antagonistic activity. In addition, this invention provides methods of screening for novel types of antiestrogen compounds that block only the indirect estrogen response and do not block estrogen action at classical estrogen response elements.

In animals and in man the balance between stimulatory and inhibitory activities of antiestrogens such as tamoxifen varies widely depending on the organ, cell or specific protein measured as an indicator of estrogenic activity. This variety of effects is difficult to reconcile with the model of antagonism of estrogen receptor (ER) activity at classical estrogen receptor elements (EREs) as described in Beato, M. *Cell,* 56: 335-344 (1989) and Klein-Hitpass, *et al., Nucleic Acids Res*., 16: 647-663 (1988).

The present invention relies, in part, on the discovery that ERs may activate transcription by interaction with another response element, the AP1 binding site, instead of binding to EREs. This AP1 mediated pathway, referred to here as the indirect estrogen response, may account for much of the agonistic properties of tamoxifen and other putative antiestrogens. A general description of the AP1 site is found in Angel & Kann, *Biochem. Biophys. Acta.,* 1072: 129-157 (1991) and Angel, *et al., Cell,* 49: 729-739 (1987).

In the methods of the invention, both the classical estrogen response elements and the indirect estrogen response may be used to provide a screening system that detects both estrogen antagonistic and agonistic activity. The methods typically comprise cultured cells that produce high levels of the human estrogen receptor. Preferred cells include MCF-7 cells or ERC1 cells described in Kushner *et al., Mol. Endocrinol*., 4:1465-1473 (1990). ERC2 and ERC3 cells as described by Webb, *et al. Mol. Endocrinol*., 6:157-167 (1993).

Other appropriate cells, including for example Ishikawa cells, and various breast cancer cells, which are known to one of skill in the art. The invention is not limited to practice in mammalian cells and may be practiced, for example in yeast and insect cells.

The cells may be modified to provide truncated or chimeric estrogen receptors as described in Berry, *et al., E.M.B.O. J., 9:* 2811-2818 (1990). These modifications may result in increased estrogen affinity and increased sensitivity of the assay.

In addition, these cells are transfected with reporter genes in which a response element (either the AP1 site or ERE) regulates expression of a reporter gene. Typically, two different reporter genes are used. One gene reports transcription induced by the classical estrogen response system, while the other gene reports transcription induced by the indirect estrogen response. The two reporter genes and response elements are typically placed in separate cells, but the methods can also be used with both constructs in the same cell.

The reporter gene for the classical estrogen response system contains an estrogen response element (ERE) upstream of the target promoter and capable of regulating that promoter. In a preferred embodiment the ERE may be the consensus estrogen response element AGGTCACAGTGACCT from the *Xenopus* vitellogenin A2 gene.

The particular ERE used in the cells is not a critical aspect of the invention and the present invention is not limited to the use of this ERE. Other EREs known to one of skill in the art can also be used. For instance, other sources of naturally occuring EREs include the B2 gene, the chicken ovalbumin gene, and the PS2 gene. Alternatively, non-naturally occuring EREs may be inserted into particular promoters. The consensus ERE from the *Xenopus* vitellogenin A2 gene is widely used for this purpose, but other EREs may be used as well.

The reporter gene for the indirect estrogen response pathway contains an AP1 site upstream of the target promoter and capable of regulating that promoter. The AP1 site is a sites that are bound by AP1 (the Jun and Fos proteins) or other members of that protein family. In a preferred embodiment, the consensus AP1 site is TGA(C/G)TCA.

One of skill would recognize that the particular AP1 site used is not a critical aspect of the invention. Any sequence capable of being bound by AP1 or members of that family and regulating a promoter is suitable. This would include promoters which encompass a naturally occuring AP1 site. Typical promoters include, but are not restricted to metalloprotease genes such as stromelysin, gelatinase, matrilysin, and the human collagenase gene.

Alternatively promoters may be constructed which contain a non-naturally occuring AP1 or related binding site. This facilitates the creation of reporter gene systems that are not typically found under the control of AP1. In addition, promoters may be constructed which contain multiple copies of the AP1 site thereby increasing the sensitivity or possibly modulating the response the reporter gene system.

The present invention is not limited to a particular reporter gene. Any gene that expresses an easily assayable product will provide a suitable indicator for the present assay. Suitable reporter genes are well known to those of skill in the art. They include, for example, bacterial chloramphenicol acetyl transferase (CAT), beta-galactosidase, or luciferase.

To screen a number of compounds for antiestrogen action, cells with high level expression of human estrogen receptors and harboring either response element and reporter genes are exposed to doses of estrogen which give half maximal induction or less. In each case this will result in induction of several to hundreds of fold depending on the levels of estrogen receptor and the particular details of the reporter construction. This will be reflected in increases of the reporter gene product, such as the CAT gene product which may be quantitated by enzymatic assay. The cells can be exposed to estrogens either growing in separate wells of a multi-well culture dish or for colorometic assay in a semi-solid nutrient matrix. The compounds to be tested are added to the culture dish wells or to small wells made in the semi-solid matrix and the effect on the estrogen induction is assayed. An antiestrogen compound will reduce or abolish the estrogen induced increase in reporter gene activity. A hypothetical pure antiestrogen will block estrogen action with both types of reporter genes and will have no ability to induce the reporter genes in the absence of estrogen. A mixed estrogen antagonist-agonist, will show some ability to induce the reporter genes, especially the reporter genes linked to AP1 site.

In another embodiment, compounds which block the indirect pathway can be used to supplement tamoxifen or other antiestrogens in the treatment or prevention of breast cancer and other diseases mediated by estrogen. These compounds function to eliminate estrogenic agonistic activity of antiestrogens. Second they may have uses by themselves. In particular, it may be advantageous to block some estrogen mediated pathological effects at indirect estrogen response elements while leaving the direct pathway active. Compounds that block the indirect pathway are useful as components of combined oral contraceptives (COC) containing estrogens and progestins. A triple COC, containing estrogens, progestins, and a blocking compound would allow estrogen, either in the formulation or endogenous to act at the classical response elements, but would block action at the indirect response elements. Thus, a triple COC functions as current COCs to prevent pregnancy, but may also provide protective effects against breast cancer.

Typically, the reporter gene linked to the AP1 site is activated, not with estrogen, but with an excess (10 times the Kd) of tamoxifen, 4-hydroxy tamoxifen, or other antiestrogen. A library of compounds is searched for candidates that block or reduce reporter gene activation by the antiestrogen. The candidates are then tested with cells containing the reporter gene for the classical pathway to confirm that they do not interfere with estrogen activation at an ERE.

Compounds identified in the assays of the invention can be used in standard pharmaceutical compositions for the treatment of cancer, as components of oral contraceptives, or any other application in which the modulation of estrogen activity is desired. The pharmaceutical compositions can be prepared and administered using methods well known in the art. The pharmaceutical compositions are generally intended for parenteral, topical, oral or local administration for prophylactic and/or therapeutic treatment. The pharmaceutical compositions can be administered in a variety of unit dosage forms depending upon the method of administration. For example, unit dosage forms suitable for oral administration include powder, tablets, pills, and capsules.

Suitable pharmaceutical formulations for use in the present invention are found in *Remington's Pharmaceutical Sciences,* Mack Publishing Company, Philadelphia, PA, 17th ed. (1985). A variety of pharmaceutical compositions comprising compounds of the present invention and pharmaceutically effective carriers can be prepared.

The following example is offered by way of illustration, not by way of limitation.

### EXAMPLE

The human collagenase gene, like other matrix metalloproteases, responds to AP1. Angel, *et al., Mol. Cell. Biol*., 7: 2256-2266 (1987). To test whether an AP1 site could confer estrogen response the collagenase promoter was fused to the bacterial CAT gene (Δcoll73) and transfected into Chinese Hamster Ovary cells that overexpress ER (ERC1) Kushner, *et al.* Mol. Endocrinol. 4: 1465-1473 (1990).

Coll73 and Coll60, previously described by Angel, P. *et al., Mol. Cell. Biol.*, 7: 2256-2266 (1987), were modified by digestion with EcoO109 and Ndel to remove an AP1 site in the backbone of pUC and were consequently designated Δ. EREΔcoll60 was prepared by ligation of a consensus ERE, AGGTCACAGTGACCT, into the HindIII site upstream of the Δcoll60 promoter. ERC transfections and electroporations were carried out as described in Kushner, *et al., Mol. Endocrinol*., 4: 1465-1473 (1990). F9 cell transfections were by calcium phosphate coprecipitation. Cells were seeded at 30% confluence upon 1.5 cmn dishes and transfected overnight with 5 mg reporter gene, 1 mg actin B-HCG.

Included in some transfections, as indicated, were 100ng of AP1 expression vectors and 1µg of ER expression vector HEO. Kumar, *et al*., *Cell,* 51: 941-951 (1987). The human cfos expression vector was BK28. Sassone-Corsi, *et al., Cell,* 54: 553-560 (1988). The human c-jun expression vector was RSVc-Jun. Turner & Tjian, *Science,* 243: 1689-1694 (1989). The cells were glycerol shocked,re-fed and hormone treated. All CAT assays were normalised to production of HCG as described in Kushner, *et al., Mol. Endocrinol.* 4: 1465-1473 (1990). Data are expressed as counts per minute of tritiated acetyl CoA converted per unit of HCG (CAT units).

Estradiol stimulated Δcoll73 ten fold (Fig. 1A), whereas a similar reporter in which the AP1 site had been removed (Δcoll60), gave reduced basal activity and no estrogen response. Substitution of a classical ERE (Klein-Hitpass, *et al., Nucl. Acids Res*. 16: 647-663 (1988)) for the AP1 site (EREΔcoll60), restored estrogen response, but not the elevated basal activity.

In F9 cells, which lack endogenous AP1 activity (Chiu, *et al. cell,* 54: 541-551 (1988) Δcoll73 failed to respond to estrogen in the presence of transiently transfected ER (Fig. 1B). Estrogen activation could be restored by cotransfecting expression vectors for AP1 proteins c-Jun and c-fos. Sassone-Corsi, P. *et al., Cell,* 54: 553-560 (1988). Turner & Tjian, *Science* 243: 1689-1694 (1989). In parallel, EREΔcoll60 was estrogen responsive, even in the absence of AP1, and Δcoll60 and remained unaffected by estrogen (data not shown). Estrogen induction of the collagenase promoter therefore required both the AP1 site and AP1 proteins.

To confirm that ER activates transcription through AP1 sites under physiological conditions, we repeated the study in MCF-7 (Fig. 2), a human breast tumor cell line in which activity of the collagenase gene family is estrogen responsive and related to tumor invasiveness. Thompson, *et al., Cancer Res.* 48: 6764-6768 (1988).

AP1ΔTK-CAT consists of the collagenase AP1 site cloned upstream of a herpes simplex virus TK promoter (-32 to +45). TK-TATA lacks any known upstream transcription factor binding sites. AP1ΔTK-CAT was constructed by ligation of an oligonucleotide spanning collagenase sequences -73 to -52 into the HindIII site upstream of a TK-CAT derivative which had been modified to remove the PUC AP1 site. AP1 TK-TATA was constructed by ligation of the same oligonucleotide into the HindIII site of a CAT vector which contained TK sequences -32 to +45 and already lacked the PUC19 AP1 site Leitmann, D.C. *et al., Mol. Cell Biol*., 12: 1352-1356 (1992). MCF-7 transfections used the same protocol as ERC cells described in Kushner, *et al., Mol. Endocrinol*., 4: 1465-1473 (1990).

Estrogen stimulated Δcoll73 activity four fold, and removal of the AP1 site abrogated estrogen responsiveness. The collagenase AP1 site also conferred estrogen response upon the herpes simplex virus TK promoter and the isolated TK TATA box, neither of which was estrogen responsive alone. Thus, the AP1 site was all that was required for estrogen response on heterologous promoters. This type of estrogen induction may represent a far more common pathway than hitherto suspected. This pathway is referred to as the indirect estrogen response.

The effects of antiestrogens on the indirect estrogen response were examined next. All cells were transfected as described in Kushner, *et al. Mol. Endocrinol*., 4: 1465-1473 (1990). Ishikawa cells, described by Holinka, *et al., J. Steroid Biochem*., 25: 781-786 (1986), were induced with 10nM PMA and 10µM forskolin prior to estrogen and antiestrogen induction.

Tamoxifen inhibits estrogen action at classical EREs by interfering with the estrogen dependent transcription activation function in the ER ligand binding domain. Berry, *et al*., *E.M.B.O. J.,* 9: 2811-2818 (1990). It sometimes shows partial agonism at classical EREs, which is attributed to activity of a cell type specific constitutive transactivation function in the amino terminus. *Id.* ICI inhibits a dimerization function (Fawell, *et al. Proc. Natl. Acad*. *Sci. U.S.A.* 87: 6883-6887 (1990)), which prevents high affinity interaction with EREs in target promoters, and does not show agonism in most assays. Wakeling, A. *J. Steroid Biiochem. Molec. Biol*., 37: 771-775 (1990).

Remarkably, both tamoxifen and ICI stimulated Δcoll73 expression five fold in ERC1 cells (Fig.3A). Neither affected Δcoll60. In contrast, EREΔcoll60 showed estrogen, but not antiestrogens response indicating that antiestrogen agonism is specific to the AP1 site. The dose response (Fig.3B) of each agonist was consistent with its affinity for the ER (Tora, *et al., E.M.B.O. J*., 8: 1981-1986 (1989) (1nM for estradiol, 10 nM for ICI and tamoxifen). We also examined antiestrogen response in cells with endogenous ER. In MCF7, tamoxifen weakly stimulated Δcoll73 expression and ICI showed little effect (Fig.3C). A human endometrial cell line (Holinka, *et al*., *J. Steroid Biochem*., 25: 781-786 which is herein incorporated by reference), estrogen, tamoxifen and ICI were equally potent in eliciting an Ap1 site-specific two fold response but did not affect the classical ERE.

The effect of transiently expressed ER mutants (Kumar, *et al. Cell,* 51: 941-951 (1987), Kumar & Chambon, *Cell*, 55: 145-155 (1988) which is herein incorporated by reference) upon Δcoll73 in CHO cells was examined to determine which ER domains mediate an indirect response. Estrogen and tamoxifen activated the collagenase promoter (Fig.4A), although both were less effective with high amounts of transiently transfected ER. ICI induction became more significant at high ER contents. This is consistent with observations that ICI reduces ER levels by reducing the half life of ER protein. Dauvois, *et al., Proc. Natl. Acad. Sci. U*.*S*.*A*., 89: 4037-44041 (1992).

The ER contains two transcriptional activation functions (Fig.4B), amino terminal constitutive (TAF-1), and carboxyl terminal estrogen dependent (TAF-2). Isolated TAF-1 (HE15) appears to give hormone independent activation of transcription and deletion of TAF-1 (HE19) reduced estrogen response and abolished antiestrogen activity. Antiestrogen activity upon Δcoll73 in CHO cells therefore reflects TAF-1 activity. This agrees with suggestions that tamoxifen cannot activate TAF-2 (Berry, *et al.* (1990)) and further implies that ICI cannot activate this function. ICI agonism may also imply that monomeric ER functions in this complex. Fawell, *et al.* (1990). Deletion of the DNA binding domain (HE11) did not reduce hormone induction suggesting that the response is independent of DNA binding and rather mediated indirectly through AP1 proteins.

In summary, indirect estrogen response is widely active, and antiestrogens are agonists of this pathway. it is possible that any of the well described agonist effects of tamoxifen reflects indirect estrogen response. Antiestrogens would have estrogenic activity on critical AP1 regulated target genes, hence growth and differentiated response, in cells in which ER and AP1 proteins could interact. Changes in AP1 during tumor progression could be particularly significant, and should be considered in models of antiestrogen resistance in breast cancer. Parker, *et al. Cancer Surveys,* 14, Growth Regulation by Nuclear Hormone Receptors. Cold Spring Harbor Laboratory Press (1992).

Although the invention is described in some detail in the above description and examples for the purposes of clarity and understanding, it will be apparent that certain changes and modifications can be made within the scope of the attached claims.

## Claims

1. A method for screening a test compound known to have antiestrogenic activity for agonistic estrogenic activity mediated through an indirect estrogen response, said method comprising:
a) providing a cell comprising an estrogen receptor and a promoter comprising an AP-1 site which regulates expression of a reporter gene;
b) contacting the cell with the test compound; and
c) detecting the expression of the reporter gene wherein enhanced expression of said reporter gene indicates that said test compound has agonistic estrogenic activity mediated through an indirect estrogen response.

2. The method of claim 1, wherein the cell is a MCF-7 cell.

3. The method of claim 1, wherein the cell is an Ishikawa cell.

4. The method of claim 1, wherein the cell is an ERC1 cell.

5. A method of anyone of claims 3 or 4 wherein the cell over-expresses the estrogen receptor.

6. A method of claim 1, wherein the reporter gene encodes beta-galactosidase.

7. A method of claim 1, wherein the reporter gene encodes bacterial chloramphenicol acetyl transferase.

8. The method of claim 1, wherein the promoter is genetically engineered to comprise an AP-1 site.

9. A method of claim 1, further comprising the steps of:
a) providing a second cell comprising an estrogen receptor and a promoter comprising a standard estrogen response element which regulates expression of a second reporter gene;
b) contacting the second cell with the test compound; and
c) detecting the expression of the second reporter gene.

10. A method of claim 9, wherein the response element is from the *Xenopus vitellogenin* A2 gene.

11. A method of claim 1, wherein the cell further comprises a promoter comprising a standard estrogen response element which regulates expression of second reporter gene.

12. A method of claim 11, wherein the response element is from the *Xenopus vitellogenin* A2 gene.

13. A method for screening a test compound for the ability to inhibit transcription through an indirect estrogen response, the method comprising:
a) providing a cell comprising an estrogen receptor and a promoter comprising an AP-1 site which regulates expression of a reporter gene;
b) contacting the cell with the test compound and a compound known to mediate an indirect estrogen response;
c) detecting the expression of the reporter gene, wherein inhibition of enhanced expression of said reporter gene produced by said compound known to have agonistic estrogenic activity mediated through an indirect estrogen response indicates that said test compound inhibits agonistic estrogenic activity mediated through an indirect estrogen response.

14. The method of claim 13, wherein the compound known to mediate an indirect estrogen response is tamoxifen.

15. The method of claim 13, wherein the compound known to mediate an indirect estrogen response is estrogen at half maximal concentration.

16. A method of claim 13, wherein the cell is a MCF-7 cell.

17. A method of claim 13, wherein the cell over-expresses the estrogen receptor.

18. A method of claim 17, wherein the cell is an ERC1 cell.

19. The method of claim 13, wherein the promoter is genetically engineered to comprise an AP1 site.

20. A method of claim 13, further comprising the steps of:
a) providing a second cell comprising an estrogen receptor and a promoter comprising a standard estrogen response element which regulates expression of a second reporter gene;
b) contacting the second cell with a test compound; and
c) detecting the expression of the second reporter gene.

21. The method of claim 20, further comprising the step of contacting the second cell with a compound known to mediate a standard estrogen response.

## Patentansprüche

1. Verfahren zum Absuchen einer Verbindung, von der bekannt ist, daß sie anti-östrogene Aktivität besitzt auf agonistische östrogene Aktivität, die durch eine indirekte Östrogenantwort vermittelt wird, wobei das Verfahren umfaßt:
a) Bereitstellen einer Zelle, umfassend einen Östrogen-Rezeptor und einen Promotor, umfassend eine AP-1-Stelle, die die Expression eines Reportergens steuert;
b) Zusammenbringen der Zelle mit der Testverbindung und
c) Nachweisen der Expression des Reportergens, wobei eine verstärkte Expression des Reportergens anzeigt, daß die Testverbindung agonistische östrogene Aktivität besitzt, die durch eine indirekte Östrogenantwort vermittelt wird

2. Verfahren nach Anspruch 1, wobei die Zelle eine MCF-7-Zelle ist.

3. Verfahren nach Anspruch 1, wobei die Zelle eine Ishikawa-Zelle ist.

4. Verfahren nach Anspruch 1, wobei die Zelle eine ERC1-Zelle ist.

5. Verfahren nach einem der Ansprüche 3 oder 4, wobei die Zelle den Östrogen-Rezeptor überexprimiert.

6. Verfahren nach Anspruch 1, wobei das Reportergen β-Galactosidase codiert.

7. Verfahren nach Anspruch 1, wobei das Reportergen bakterielle Chloramphenicol-acetyl-Transferase codiert.

8. Verfahren nach Anspruch 1, wobei der Promotor gentechnisch verändert ist, um eine AP-1-Stelle zu enthalten.

9. Verfahren nach Anspruch 1, umfassend ferner die folgenden Stufen:
a) Bereitstellen einer zweiten Zelle, umfassend Östrogen-Rezeptor und einen Promotor, umfassend ein Standard-Östrogenantwort-Element, das die Expression eines zweiten Reportergens steuert;
b) Zusammenbringen der zweiten Zelle mit der Testverbindung und
c) Nachweisen der Expression des zweiten Reportergens.

10. Verfahren nach Anspruch 9, wobei das Antwortelement von dem Xenopus vitellogenin A2 Gen stammt.

11. Verfahren nach Anspruch 1, wobei die Zelle femer einen Promotor umfaßt, der ein Standard-Östrogenantwort-Element umfaßt, das die Expression des zweiten Reportergens steuert.

12. Verfahren nach Anspruch 11, wobei das Antwortelement von dem Xenopus vitellogenin A2 Gen stammt.

13. Verfahren zum Absuchen einer Verbindung, auf ihre Fähigkeit, die Transcription durch eine indirekte Östrogenantwort zu hemmen, wobei das Verfahren umfaßt:
a) Bereitstellen einer Zelle, umfassend einen Östrogen-Rezeptor und einen Promotor, umfassend eine AP-1-Stelle, die die Expression eines Reportergens steuert;
b) Zusammenbringen der Zelle mit der Testverbindung und einer Verbindung, von der bekannt ist, daß sie eine indirekte Östrogenantwort vermittelt,
c) Nachweisen der Expression des Reportergens, wobei die Hemmung der verstärkten Expression des Reportergens, das durch die Verbindung gebildet worden ist, von der bekannt ist, daß sie durch eine indirekte Östrogenantwort vermittelte agonistische östrogene Aktivität besitzt, anzeigt, daß die Testverbindung agonistische östrogene Aktivität, die durch eine indirekte Östrogenantwort vermittelt wird hemmt.

14. Verfahren nach Anspruch 13, wobei die Verbindung, von der bekannt ist, daß sie eine indirekte Östrogenantwort vermittelt, Tamoxifen ist.

15. Verfahren nach Anspruch 13, wobei die Verbindung, von der bekannt ist, daß sie eine indirekte Östrogenantwort vermittelt, Östrogen in halber maximaler Konzentration ist.

16. Verfahren nach Anspruch 13, wobei die Zelle eine MCF-7-Zelle ist.

17. Verfahren nach Anspruch 13, wobei die Zelle den Östrogen-Rezeptor überexprimiert.

18. Verfahren nach Anspruch 13, wobei die Zelle eine ERC1-Zelle ist.

19. Verfahren nach Anspruch 13, wobei der Promotor gentechnisch verändert ist, um eine AP-1-Stelle zu enthalten.

20. Verfahren nach Anspruch 13, umfassend ferner die folgenden Stufen:
a) Bereitstellen einer Zelle, umfassend Östrogen-Rezeptor und einen Promotor, umfassend ein Standard-Östrogenantwort-Element, das die Expression eines zweiten Reportergens steuert;
b) Zusammenbringen der zweiten Zelle mit der Testverbindung und
c) Nachweisen der Expression des zweiten Reportergens.

21. Verfahren nach Anspruch 20, umfassend femer die Stufe des Zusammenbringens der zweiten Zelle mit einer Verbindung, von der bekannt ist, daß sie eine Standard-Östrogenantwort vermittelt.

## Revendications

1. Procédé pour le criblage d'un composé d'essai connu pour avoir une activité anti-oestrogénique pour une activité oestrogénique agoniste à médiation par une réponse par oestrogène indirecte, ledit procédé comprenant:
a) la fourniture d'une cellule comprenant un récepteur d'oestrogène et un promoteur comprenant un site AP-1 qui régule l'expression d'un gène reporter;
b) la mise en contact de la cellule avec le composé d'essai; et
c) la détection de l'expression du gène reporter, tandis que l'expression renforcée dudit gène reporter indique que ledit composé d'essai a une activité oestrogénique agoniste à médiation par une réponse par oestrogène indirecte .

2. Procédé selon la revendication 1, dans lequel la cellule est une cellule MCF-7.

3. Procédé selon la revendication 1, dans lequel la cellule est une cellule de Ishikawa.

4. Procédé selon la revendication 1, dans lequel la cellule est une cellule ERC1.

5. Procédé selon l'une quelconque des revendications 3 ou 4, dans lequel la cellule surexprime le récepteur d'oestrogène.

6. Procédé selon la revendication 1, dans lequel le gène reporter code pour la bêta-galactosidase.

7. Procédé selon la revendication 1, dans lequel le gène reporter code pour la chloramphénicol acétyl transférase bactérienne.

8. Procédé selon la revendication 1, dans lequel le promoteur est manipulé génétiquement pour comporter un site AP-1.

9. Procédé selon la revendication 1, comprenant en outre les étapes de:
a) fourniture d'une deuxième cellule comprenant un récepteur d'oestrogène et un promoteur comprenant un élément de réponse par oestrogène standard qui régule l'expression d'un deuxième gène reporter;
b) la mise en contact de la deuxième cellule avec le composé d'essai; et
c) la détection de l'expression du deuxième gène reporter.

10. Procédé selon la revendication 9, dans lequel l'élément de réponse est issu du gène A2 de *Xenopus vitellogenin*.

11. Procédé selon la revendication 1, dans lequel la cellule comprend en outre un promoteur comprenant un élément de réponse par oestrogène standard qui régule l'expression d'un deuxième gène reporter.

12. Procédé selon la revendication 11, dans lequel l'élément de réponse est issu du gène A2 de *Xenopus vitellogenin.*

13. Procédé pour le criblage d'un composé d'essai quant à l'aptitude à inhiber la transcription par l'intermédiaire d'une réponse par oestrogène indirecte, le procédé comprenant:
a) la fourniture d'une cellule comprenant un récepteur d'oestrogène et un promoteur comprenant un site AP-1 qui régule l'expression d'un gène reporter;
b) la mise en contact de la cellule avec le composé d'essai et un composé connu pour assurer la médiation d'une réponse par oestrogène indirecte;
c) la détection de l'expression du gène reporter, tandis que l'inhibition de l'expression renforcée du dit gène reporter produite par ledit composé connu pour avoir une activité oestrogène agoniste à médiation par une réponse par oestrogène indirecte indique que ledit composé d'essai inhibe l'activité oestrogénique agoniste à médiation par une réponse par oestrogène indirecte.

14. Procédé selon la revendication 13, dans lequel le composé connu pour assurer la médiation d'une réponse par oestrogène indirecte est le tamoxifène.

15. Procédé selon la revendication 13, dans lequel le composé connu pour assurer la médiation d'une réponse par oestrogène indirecte est l'oestrogène à la concentration moitié du maximum.

16. Procédé selon la revendication 13, dans lequel la cellule est une cellule MCF-7.

17. Procédé selon la revendication 13, dans lequel la cellule surexprime le récepteur d'oestrogène.

18. Procédé selon la revendication 17, dans lequel la cellule est une cellule ERC1.

19. Procédé selon la revendication 13, dans lequel le promoteur est manipulé génétiquement pour comporter un site AP1.

20. Procédé selon la revendication 13, comprenant en outre les étapes de:
a) fourniture d'une deuxième cellule comprenant un récepteur d'oestrogène et un promoteur comprenant un élément de réponse par oestrogène standard qui régule l'expression d'un deuxième gène reporter;
b) la mise en contact de la deuxième cellule avec un composé d'essai; et
c) la détection de l'expression du deuxième gène reporter.

21. Procédé selon la revendication 20, comprenant en outre l'étape de mise en contact de la deuxième cellule avec un composé connu pour assurer la médiation d'une réponse par oestrogène standard.
